# EUROPEAN PATENT APPLICATION

(11) **EP 2 592 074 A1**
(43) Date of publication of application: **15.05.2013**
(21) Application number: 10782170.4
(22) Date of filing: 16.07.2010
(51) Int. Cl.: C07D 335/16, C07D 409/14, C08F 2/48

(54) **THIOXANTHONE-4-CARBOXYLATES, THEIR PREPARATION METHODS, PHOTOINITIATOR COMPOSITIONS AND USES THEREOF**

(30) Priority: 06.07.2010 CN 201010218422
(71) Applicant: Tianjin Jiuri Chemical Co., Ltd., Tianjin 300384 (CN)
(72) Inventor: ZHAO, Guofeng, Tianjin 300384 (CN)
(74) Representative: Larcher, Dominique
(86) International application number: PCT/CN2010/075209
(87) International publication number: WO 2012/003644

(57) **Abstract**

The present invention relates to thioxanthone-4-carboxylates, a method for the preparation of the compounds, photoinitiator compositions and use of them as photoinitiators. The compounds of the invention have a structure according to Formula ( I ) below: 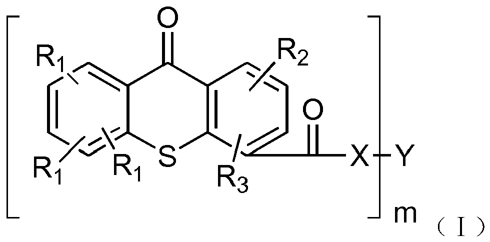 in which:
each R₁ is independently selected from the group consisting of H, halogen, C₁-C₁₂ alkyl, C₃-C₆ cycloalkyl or C₁-C₁₂ alkoxy;
R₂ and R₃ are independently selected from the group consisting of H, halogen, C₁-C₁₈ alkyl, C₃-C₆ cycloalkyl or C₁-C₁₈ alkoxy;
Y is independently selected from the group consisting of C₁-C₁₈ alkoxy or small molecular compounds containing 2-6 hydroxyl group;
m is a number from 1 to 6; and
X is 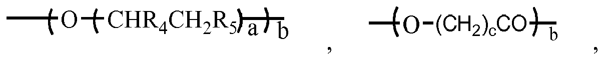 or

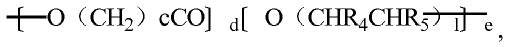

wherein R₄ and R₅ are each independently selected from the group consisting of H, halogen, C₁-C₆ alkyl; a is a number from 1 to 6; b, d, e respectively represent a number from 1 to 25, c is a number from 1 to 5. The invention provides the compounds with the characters of low extraction, low migration and low volatility and use of them as photoinitiators in photo-polymerization, or package materials of medicine or food. As well, the raw materials of the invention are easily got, and the process of the invention is simple, so thioxanthone-4-carboxylate derivatives are suitable for industrial production.

## Description

### 1. Background Art

The present invention relates to thioxanthone-4-carboxylate derivatives, a method for the preparation of the compounds, photoinitiator compositions and use of them as photoinitiators in photo-polymerization, or package materials of medicine or food.

It is known that thioxanthone derivatives are important intermediates in the field of fine chemicals and drugs, sensitizers for photo-polymerization of ethylenically unsaturated compounds.

Currently, it is also known that thioxanthone derivatives widely used and commercially available are mainly 2-chlorothioxanthen-9-one (CTX), 2,4-diethyl-9H-thioxanthen-9-one (DETX), isopropyl thioxanthone (ITX). CTX, DETX and ITX are small molecules, and have some toxicity. when they are used as the photoinitiator in coatings, inks, package materials of medicine or food, the unreacted thioxanthones (CTX, ITX) will migrate to the polymer film surface, and result in poison. So thioxanthone derivatives, which are used in coatings, inks, medicine or food, must have good curing rate, odorless and good solubility, and the surface of curing membrane must be solid, smooth, uniform, while they should have very low mobility and low solvent extraction rate. The analysis of unreacted thioxanthones extracted from optical polymer film has a certain relationship with extracting solvent and extracting method, and common extracting solvent in analyzing package materials of drinks or food is 10% ethanol solution, 3% acetic acid solution or 95% ethanol solution.

WO03/033492 discloses a series of multi-functional thioxanthone photoinitiators, prepared from 2-hydroxythioxanthone and varies of polyethers. Such photoinitiators have low extraction, low migration and low volatility, but the synthesis of 2-hydroxythioxanthone will produce severe pollution. To solve this problem, Great Lakes Chemical Corporation have made improvement on the synthesis of the materials to eliminate the pollution of phenol in the patent US20040059133, but the difficulty of the treatment with large quantities of sulfuric acid occurs.

US4385182 discloses thioxanthone-1-carboxylic acid, esters and their derivatives. The patent has described their performance as photoinitiator, such as the irradiation time, curing rate, and has explored their role in a series of optical polymer system. US4505794 discloses thioxanthone-1-carboxylic acid, thioxanthone-3-carboxylic acid, esters, their derivatives and photopolymerization properties.

US6025408 and US4594400 disclose macromolecular photoinitiators with thioxanthonecarboxylic acid, esters and their derivatives as side chains prepared by the polymerization of polymeric monomers of thioxanthone-1-carboxylic acid derivatives with other monomers. Such macromolecular photoinitiators have great difficulties in actual application due to their high viscosity, although they have lower extraction, lower migration and lower volatility than previous photoinitiators. Similar compounds are disclosed by Pouliquen et al. [Macromolecules, Vol.28, 8028-8034(1995)], WO97/49664 and ES2015341.

EP0167489 discloses liquid thioxanthone photoinitiators with the end connecting to thioxanthones obtained by condensation reaction of thioxanthonecarboxylic acids and polyethers. But the patent has not mentioned if the compounds have the abilities of low extraction, low migration and low volatility.

CN200410093977 discloses thioxanthone-2-carboxylates macromolecular photoinitiators which have low extraction, low migration and low volatility. But the high cost of the raw materials restricts the application of these compounds.

### 2. Summary of the invention

The invention provides thioxanthone-4-carboxylate derivatives and preparation of the compounds. These compounds have shown high activity as photoinitiators with low extraction, low migration and low volatility after polymerization.

The invention also provides photoinitiator compositions and use of them as photoinitiators in photo-polymerization, or package materials of coatings, printing ink, medicine or food because of low cost, high activity, low extraction, low migration and low volatility. Thioxanthone-4-carboxylate derivatives overcome the defects of previous photoinitiators and are suitable for industrial production.

The compounds of the invention have a structure according to Formula ( I ) below: 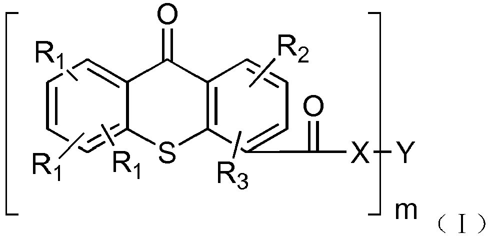 in which:
each R₁ is independently selected from the group consisting of H, halogen, C₁-C₁₂ alkyl, C₃-C₆ cycloalkyl or C₁-C₁₂ alkoxy;
R₂ and R₃ are independently selected from the group consisting of H, halogen, C₁-C₁₈ alkyl, C₃-C₆ cycloalkyl or C₁-C₁₈ alkoxy;
Y is independently selected from the group consisting of C₁-C₁₈ alkoxy or small molecular compounds containing 2-6 hydroxyl groups;
m is a number from 1 to 6; and
X is 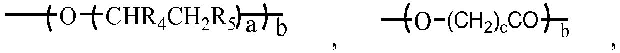 or 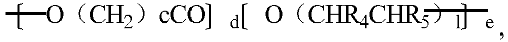
wherein: R₄ and R₅ are each independently selected from the group consisting of H, halogen, C₁-C₆ alkyl; a is a number from 1 to 6; b, d, e respectively represent a number from 1 to 25, c is a number from 1 to 5.

Preferred compounds of Formula I are those in which:
R₁, R₂ and R₃ are each independently selected from the group consisting of H;
Y is independently selected from the group consisting of C₁-C₁₈ alkoxy or small molecular compounds containing 2-5 hydroxyl group, for example: (̵O-CH₂CH₂O-)̵ (̵O-CH₂CH₂CH₂CH₂O)̵ 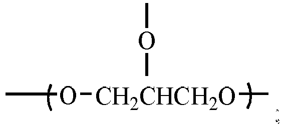
m is the number of 1;
X is independently selected from the group consisting of polyethers, polyesters, or their copolymers as follows: 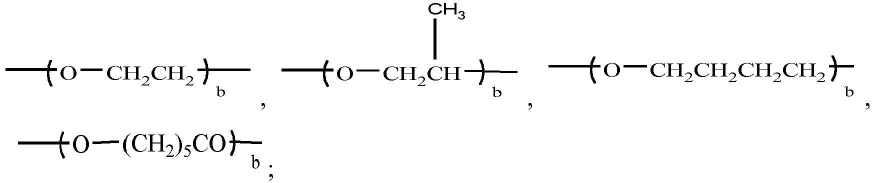 wherein: R₄ and R₅ are each independently selected from the group consisting of H, methyl, ethyl; a is a number from 1 to 3; b is a number from 1 to 18, more preferably from 1 to 10; d, e respectively represent a number from 1 to 18.

Exemplary compounds in accordance with Formula ( I ) include but are not limited to:

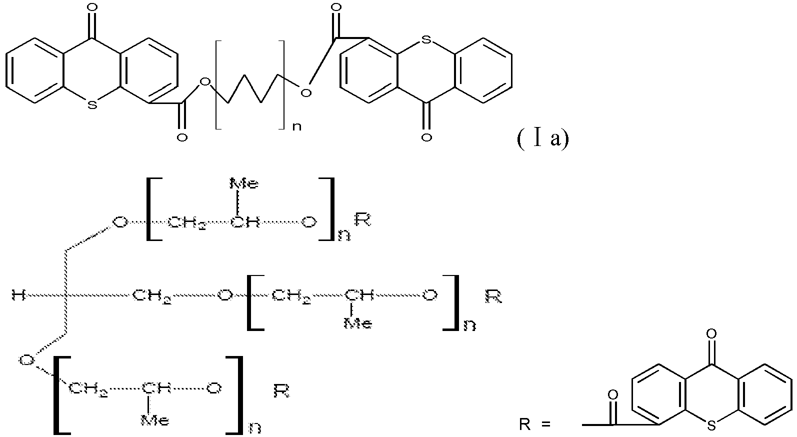

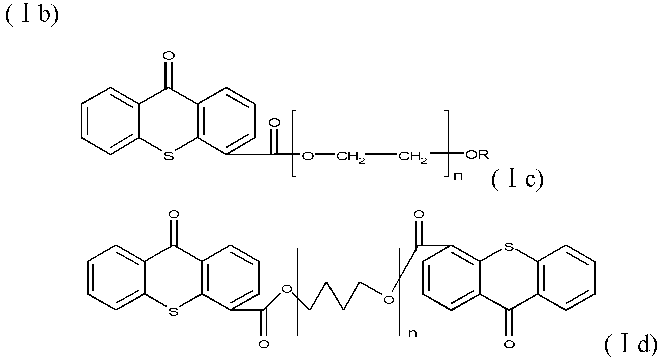

The preparation of the compounds of the invention is provided as follows:
(1) 2-Chlorobenzoic acid, thiosalicylic acid, react with base (e.g. sodium hydroxide) in organic solvents (e.g. dimethylformamide) to generate 2-(2-carboxyphenyl)sulfanylbenzoic acid which can be cyclized by 98% sulfuric acid to give the compound of thioxanthone-4-carboxylic acid. Thioxanthone-4-carboxylic acid react with thionyl chloride under reflux, and then react with alcohols to generate thioxanthone-4-carboxylate derivatives.
   Reaction formulas are as follows: 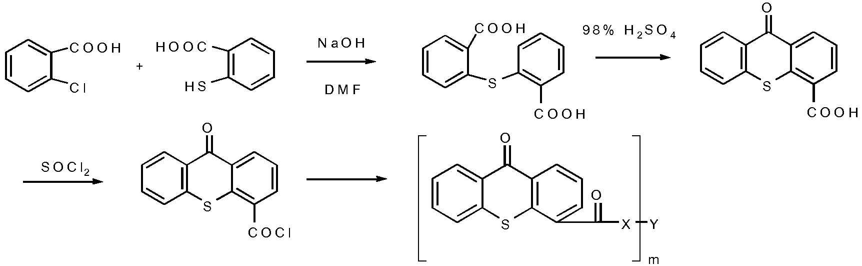 wherein: the weight ratio between 2-chlorobenzoic acid and thiosalicylic acid is from 1:1 to 2:1; the weight ratio between 2-chlorobenzoic acid and sodium hydroxide is from 2:1 to 5:1; the weight ratio between 2-(2-carboxyphenyl)sulfanylbenzoic acid and 98% sulfuric acid is from 2:1 to 7:1; the organic solvents of the invention can be dimethylformamide, hexamethyl phosphoryl triamide, 2-pyrrolidone, dimethyl sulfoxide, benzene or more of them; the base of the invention can be one or more organic bases, such as sodium hydroxide, potassium hydroxide, lithium hydroxide, sodium methoxide, sodium ethoxide or sodium hydride. Or
(2) Thioxanthone-4-carboxylic acid is prepared by the method above, and then react with lower alcohols (e.g. methanol) to generate thioxanthone-4-carboxylates. Thioxanthone-4-carboxylates are taking part in transesterification with the corresponding alcohol (e.g. polyethylene glycol-200) in the presence of catalyst. Reaction formulas are as follows: 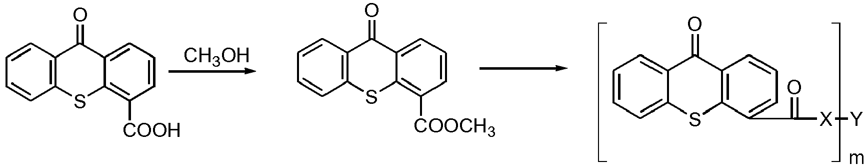 wherein: the catalyst can be acids (e.g. 98% sulfuric acid, methanesulfonic acid, benzenesulfonic acid, concentrated hydrochloric acid, etc) or organotins catalyst (e.g. n-butyltin hydroxide oxide, dibutyltin oxide, etc); the weight ratio between thioxanthone-4-carboxylate and catalyst is from 20:1 to 70:1.

Other substituted thioxanthone-4-carboxylic acids or thioxanthone-4-carboxylates can be prepared according to the methods of the invention through making ordinary skill improvement in this area.

In another embodiment of the invention, photopolymerizable compositions which include a compound of Formula ( I ) above as a photoinitiator are provided.

Generally, the compositions of the invention include a compound of Formula ( I ) and one or more organic amines. The weight ratio between organic amines and the compound of Formula ( I ) is from 1:0.8 to 1:1.5. Examples are: triethylamine, methyldiethanolamine, N,N-dimethyl ethanolamine, N,N-diethylethanolamine or N,N-dimethylaminoethyl acrylate.

The photoinitiation system of the invention includes photopolymerizable compositions above, one or more ethylenically unsaturated compounds in amounts between about 0.1 and about 15 percent by weight and additives.

Ethylenically unsaturated compounds used can be prepolymers, epoxy oligomers, amino polymers, for example, polyurethane prepolymers, polyether polyisocyanate prepolymers, epoxy acrylate oligomers, epoxy polystyrene oligomers, amino acrylate polymers or p-amino benzaldehyde polymers.

The additives include silk screen inks, offset printing inks, flexo inks, wood coatings and so on.

The photoinitiation systems of the invention, especially printing inks, will produce cured compounds with properties of paint, varnish, enamel, pigment or ink by photopolymerization.

The photoinitiation systems of the invention can be used as oily or water borne coating agents.

The invention also relate to the use of photoinitiator compositions which include a compound o f Formula (I) above as photoinitiators. The available photopolymerization ingredients of the invention mean the cured compositions after exposure to radiation.

The available photopolymerization ingredients of the invention also include photoinitiator or synergist as a component that acts as hydrogen atom donor. The photoinitiator or synergist, which is usually selected from alcohol, amine or ester, can improve the efficiency and speed of polymerization.

The available photopolymerization ingredients of the invention can be painted onto a basal surface by means of general technology and equipments. The compositions can be painted continuously or discontinuously to form the biofilm.

The available photopolymerization ingredients of the invention can be used in any known photopolymerization, including solid adhesive that produces cured compounds with properties of paint, varnish, enamel, pigment or ink by photopolymerization.

The invention provides thioxanthone-4-carboxylate derivatives which have shown high activity as photoinitiators with low extraction, low migration and low volatility. These compounds will not migrate to the package surface and avoid being extracted to the goods after photopolymerization. The invention overcomes the difficulties of separating products from raw materials, recycling, solubility and easily condensing or salting out. So the compounds are very suitable for using in the packing of foods, medicine or others, and also can be used in painting or inks. As well, the raw materials of the invention are easily acquired and the process of the invention is simple. So thioxanthone-4-carboxylate derivatives are suitable for industrial production.

### 3. Embodiments

The present invention will be further illustrated by the following non-limiting examples.

### EXAMPLE 1 : Preparation of 2-(2-carboxyphenyl)sulfanylbenzoic acid

In order, 47.0 grams (0.30 mol) of 2-chlorobenzoic acid, 46.2 grams (0.30 mol) of thiosalicylic acid, 100 mL of dimethylformamide, 13.2 grams (0.33 mol) of sodium hydroxide were combined in a 250 mL flask equipped with mechanical stirrer. The mixture was well stirred under reflux for 5 hours. After cooling, the mixture was evaporated. After the addition of 100 mL of water, the residue was added dropwise 50 mL of dilute hydrochloric acid with stirring and adjusted to pH=1. The product was filtered off. After recrystallisation from 50 mL of methanol, 74.0 grams (90% of theory) of 2-(2-carboxyphenyl)sulfanylbenzoic acid was obtained. The purity is 98.5%.

2-(2-Carboxyphenyl)sulfanylbenzoic acid. Pale yellow cryst; m.p. 229-233°C;'H-NMR (300 MHz, DMSO):δ 7.10 (2H, m), 7.38 (2H, m), 7.46 (2H, m), 7.84 (2H, m), 13.12 (2H, s).

### EXAMPLE 2 : Preparation of thioxanthone-4-carboxylic acid

In order, 40.0 grams (0.15 mol) of 2-(2-carboxyphenyl)sulfanylbenzoic acid, 3.0 grams of 98% sulfuric acid, 30 mL of xylene were combined in a 250 mL flask equipped with mechanical stirrer. The mixture was well stirred under reflux for 5 hours. After cooling, the mixture was added 50 mL of water, and the product was filtered off. After recrystallisation from 30 mL of methanol, 33.6 grams (90% of theory) of thioxanthone-4-carboxylic acid was obtained. The purity is 99.5%.

Thioxanthone-4-carboxylic acid. Yellow powder; m.p. 353 °C;¹H-NMR (300 MHz, DMSO) :δ 7.10-7.80 (6H, m), 8.40 (1H, m), 12.81 (1H, s).

### EXAMPLE 3 :Preparation of thioxanthone-4-carboxylate

In order, 25.6 grams (0.10 mol) of thioxanthone-4-carboxylic acid, 50 mL of thionyl chloride were combined in a 100 mL flask equipped with mechanical stirrer. The mixture was well stirred under reflux for 5 hours. After cooling, the mixture was evaporated and added 50 mL of toluene and 5 grams of absolute alcohol below 50 °C. The mixture was well stirred under reflux for 2 hours. After slowly cooling, the product was filtered off and 27.0 grams (95% of theory) of thioxanthone-4-carboxylate was obtained. The purity is 99.2%.

Thioxanthone-4-carboxylate. Pale yellow cryst; m.p. 149-150 °C;¹H-NMR (300 MHz, DMSO) :δ 1.30 (3H, t, *J*=7.2), 4.23 (2H, q, *J*=7.2), 7.10-7.71 (6H, m), 8.23 (1H, m).

### EXAMPLE 4 :Preparation of thioxanthone-4-carboxylate

In order, 27.4 grams (0.10 mol) of 2-(2-carboxyphenyl)sulfanylbenzoic acid, 4.0 grams of 98% sulfuric acid and 30 mL of xylene were combined in a 100 mL flask equipped with mechanical stirrer. The mixture was well stirred under reflux for 5 hours. After cooling, the mixture was added 8 grams of absolute alcohol. The mixture was well stirred under reflux for 5 hours. After slowly cooling, the product was filtered off and 25.5 grams (90% of theory) of thioxanthone-4-carboxylate was obtained. The purity is 99.5%.

### EXAMPLE 5 : Preparation of thioxanthone-4-carboxylicpolytetrahydrofuran-250-diester

In order, 25.6 grams (0.10 mol) of thioxanthone-4-carboxylic acid, 50 mL of thionyl chloride were combined in a 100 mL flask equipped with mechanical stirrer. The mixture was well stirred under reflux for 5 hours. The excess of thionyl chloride was evaporated off in vacuum. The residue was added 50 mL of toluene and dropwise 12.4 grams of polytetrahydrofuran-250 below 50 °C. The mixture was well stirred under reflux for 2 hours. After slowly cooling, the product was filtered off and 32.6 grams (90% of theory) of thioxanthone-4-carboxylicpolytetrahydrofuran-250-diester was obtained. The purity is 96%.

Thioxanthone-4-carboxylicpolytetrahydrofuran-250-diester. Pale yellow powder; m.p. 82-85 °C; MS (m/z): 639.15, 712.34, 728.23, 872.35.

### EXAMPLE 6 :Preparation of thioxanthone-4-carboxylicpolytetrahydrofuran-250-diester

In order, 28.4 grams (0.10 mol) of thioxanthone-4-carboxylate, 50 mL of 1,2-dichlorobenzene, 0.5 grams of n-butyltin hydroxide oxide and 12.5 grams of polytetrahydrofuran-250 were combined in a 100 mL flask equipped with mechanical stirrer. The mixture was well stirred at 150 °C for 4 hours with distilling the produced ethanol. After cooling, the catalyst was filtered out and the mixture was evaporated to remove the solvent. By adding 30 mL of methanol, the product was filtered off and 34.5 grams (95% of theory) of thioxanthone-4-carboxylicpolytetrahydrofuran-250-diester was obtained. The purity is 96%.

### EXAMPLE 7 : Preparation of thioxanthone-4-carboxylicpolyethylene glycol-200-diester

In order, 25.6 grams (0.10 mol) of thioxanthone-4-carboxylic acid, 50 mL of thionyl chloride were combined in a 100 mL flask equipped with mechanical stirrer. The mixture was well stirred under reflux for 5 hours. The excess of thionyl chloride was evaporated off in vacuum. The residue was added 50 mL of toluene and dropwise 9.9 grams of polyethylene glycol-200 below 50 °C. The mixture was well stirred under reflux for 2 hours. After slowly cooling, the product was filtered off and 30.4 grams (90% of theory) of thioxanthone-4-carboxylicpolyethylene glycol-200-diester was obtained. The purity is 95.8%.

Thioxanthone-4-carboxylicpolyethylene glycol-200-diester. Pale yellow powder; m.p. 80-84 °C; MS (m/z): 626.30, 670.84, 714.47.

### EXAMPLE 8 :Preparation of thioxanthone-4-carboxylicpolyethylene glycol-200-diester

In order, 28.4 grams (0.10 mol) of thioxanthone-4-carboxylate, 50 mL of 1,2-dichlorobenzene, 0.5 grams of n-butyltin hydroxide oxide and 10 grams of polyethylene glycol-200 were combined in a 100 mL flask equipped with mechanical stirrer. The mixture was well stirred at 150 °C for 4 hours with distilling the produced ethanol. After cooling, the catalyst was filtered out and the mixture was evaporated to remove the solvent. By adding 30 mL of methanol, the product was filtered off and 32.1 grams (95% of theory) of thioxanthone-4-carboxylicpolyethylene glycol-200-diester was obtained. The purity is 96.5%.

### EXAMPLE 9 :Preparation of thioxanthone-4-carboxylicpolyethylene glycol-300-diester

In order, 25.6 grams (0.10 mol) of thioxanthone-4-carboxylic acid, 50 mL of thionyl chloride were combined in a 100 mL flask equipped with mechanical stirrer. The mixture was well stirred under reflux for 5 hours. The excess of thionyl chloride was evaporated off in vacuum. The residue was added 50 mL of toluene and dropwise 14.8 grams of polyethylene glycol-300 below 50 °C. The mixture was well stirred under reflux for 2 hours. After slowly cooling, the product was filtered off and 34.9 grams (90% of theory) of thioxanthone-4-carboxylicpolyethylene glycol-300-diester was obtained. The purity is 96%.

Thioxanthone-4-carboxylicpolyethylene glycol-300-diester. Pale yellow powder; m.p. 50-55 °C; MS(m/z): 714.60, 758.34, 802.24, 846.13.

### EXAMPLE 10 : Preparation of thioxanthone-4-carboxylicpolyethylene glycol-300-diester

In order, 28.4 grams (0.10 mol) of thioxanthone-4-carboxylate, 50 mL of 1,2-dichlorobenzene, 0.5 grams of n-butyltin hydroxide oxide and 10 grams of polyethylene glycol-300 were combined in a 100 mL flask equipped with mechanical stirrer. The mixture was well stirred at 150 °C for 4 hours with distilling the produced ethanol. After cooling, the catalyst was filtered out and the mixture was evaporated to remove the solvent. By adding 30 mL of methanol, the product was filtered off and 36.8 grams (95% of theory) of thioxanthone-4-carboxylicpolyethylene glycol-300-diester was obtained. The purity is 96.5%.

### EXAMPLE 11 : Performance Evaluation of photoinitiator I a

Experiment formula of the photoinitiator system is as follows:

| | |
|---|---|
| Epoxy acrylic resin (M.W. 700-800) | 20%wt |
| Pentaerythritol triacrylate | 18%wt |
| Photoinitiator I a or ITX | 1.5%wt |
| Triethanolamine | 4.5%wt |
| Benzyladenine /Urethane oligomer | 34%wt |
| Paint | 12%wt |

Experiment and evaluation:
Paint the mixture above onto the glass to form film, and the curing of film is finished through irradiating with standard mercury lamp. After being irradiated at speed of 20 m/min, it is found that the film is solid, smooth and uniform. The film strength is 13 MPa and impact energy by pendulum is 38 Kj/m².

### EXAMPLE 12 :Analysis of the gas volatilization of photoinitiator I a

20 cm² of the sample in example 6 and 1 grams of activated charcoal on watch-glass, was placed into the oven and heated at 180 °C for 10 min. After cooling, the sample was removed and the activated charcoal was extracted with tetrahydrofuran.
Then the content of photoinitiator I a or ITX measured by high performance liquid chromatography and the results are listed in table 1.

**Table 1 The results of the gas volatilization**

| Photoinitiator | Adsorbent by activated charcoal ( mg/m²) |
|---|---|
| ITX | 6.1 |
| Ia | <0.05 |

The results show that the gas volatilization of photoinitiator I a is very low and that of ITX is high.

### EXAMPLE 13 :Analysis of migration of photoinitiator I a

15 x 15 cm² of samples in example 6 and filter paper of of diameter 10 cm are placed between two stainless foils, and kept under the pressure of 5 t for 72 hours. Then test the contents of photoinitiator I a and ITX by HPLC. The results are listed in table 2.

**Table 2 The results of migration content by contacting**

| Photoinitiator | Adsorbent by filter paper (mg/m²) |
|---|---|
| ITX | 1.58 |
| Ia | 0.04 |

The results show that photoinitiator I a has low migration by contacting.

## Claims

1. A compound of the formula I 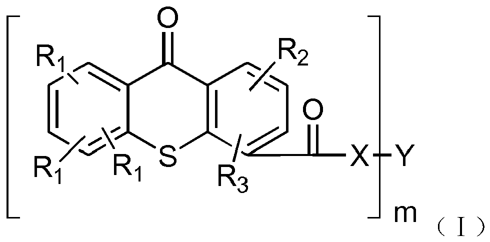 in which:
each R₁ is independently selected from the group consisting of H, halogen, C₁-C₁₂ alkyl, C₃-C₆ cycloalkyl or C₁-C₁₂ alkoxy;
R₂ and R₃ are independently selected from the group consisting of H, halogen, C₁-C₁₈ alkyl, C₃-C₆ cycloalkyl or C₁-C₁₈ alkoxy;
Y is independently selected from the group consisting of C₁-C₁₈ alkoxy or small molecular compounds containing 2-6 hydroxyl groups;
m is a number from 1 to 6; and
X is independently selected from the group consisting of polyethers, polyesters, or their copolymers: 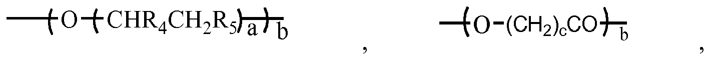 or 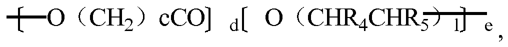
wherein R₄ and R₅ are each independently selected from the group consisting of H, halogen, C₁-C₆ alkyl; a is a number from 1 to 6; b, d, e respectively represent a number from 1 to 25, c is a number from 1 to 5.

2. A compound of the formula I according to claim 1, in which preferly R₁, R₂ and R₃ are each independently selected from the group consisting of H.

3. A compound of the formula I according to claim 1, in which X is independently selected from the group consisting of polyethers, polyesters, or their copolymers, wherein preferly R₄ and R₅ are each independently selected from the group consisting of H, methyl, ethyl.

4. A compound of the formula I according to claim 1, in which preferly a is a number from 1 to 3; b, d, e respectively represent a number from 1 to 18.

5. A compound of the formula I according to claim 1, in which X is 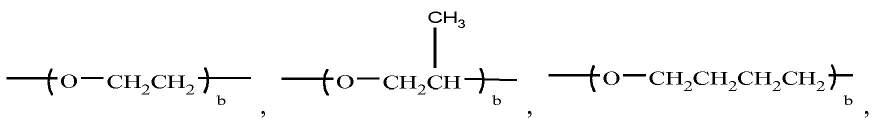 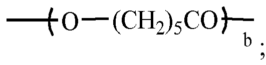 wherein preferly b is a number from 1 to 10.

6. A compound of the formula I according to claim 1, in which Y is independently selected from the group consisting of C₁-C₁₈ alkoxy or small molecular compounds preferly containing 2-5 hydroxyl groups; preferly m is a number of 1.

7. A process for prepararing the compounds of formula I according to claim 1, comprising:
2-Chlorobenzoic acid, thiosalicylic acid, react with base in organic solvents to generate 2-(2-carboxyphenyl)sulfanylbenzoic acid which can be cyclized by concentrated sulfuric acid to give the compound of thioxanthone-4-carboxylic acid, Thioxanthone-4-carboxylic acid react with thionyl chloride under reflux, and then react with alcohols to generate thioxanthone-4-carboxylate derivatives; or Thioxanthone-4-carboxylic acid is prepared by the method above, and then react with lower alcohols to generate thioxanthone-4-carboxylates; Thioxanthone-4-carboxylates are taking part in transesterification with the corresponding alcohols by the catalyst,
wherein: the catalyst can be acid compounds: concentrated sulfuric acid, methanesulfonic acid, benzenesulfonic acid, concentrated hydrochloric acid or organotins catalyst: n-butyltin hydroxide oxide, dibutyltin oxide.

8. A process for prepararing the compounds of formula I according to claim 7, **characterized in that** the organic solvents of the invention can be dimethylformamide, hexamethyl phosphoryl triamide, 2-pyrrolidone, dimethyl sulfoxide, benzene or more of them.

9. A process for prepararing the compounds of formula I according to claim 7, **characterized in that** the weight ratio between 2-chlorobenzoic acid and thiosalicylic acid is from 1:1 to 2:1.

10. A process for prepararing the compounds of formula I according to claim 7, **characterized in that** the base of the invention can be one or more organic bases:
sodium hydroxide, potassium hydroxide, lithium hydroxide, sodium methoxide, sodium ethoxide or sodium hydride.

11. A process for prepararing the compounds of formula I according to claim 7, **characterized in that** the weight ratio between 2-chlorobenzoic acid and sodium hydroxide is from 2:1 to 5:1.

12. A process for prepararing the compounds of formula I according to claim 7, **characterized in that** the weight ratio between 2-(2-carboxyphenyl)sulfanylbenzoic acid and 98% sulfuric acid is from 2:1 to 7:1.

13. A process for prepararing the compounds of formula I according to claim 7, **characterized in that** the weight ratio between thioxanthone-4-carboxylate and catalyst is from 20:1 to 70:1.

14. A composition of the photoinitiation system comprising a compound of Formula ( I ) according to claim 1 and one or more organic amines of triethylamine, methyldiethanolamine, N,N-dimethyl ethanolamine, N,N-diethylethanolamine or N,N-dimethylaminoethyl acrylate, wherein the weight ratio between organic amines and the compound of Formula ( I ) is from 1:0.8 to 1:1.5..

15. A photoinitiation system comprising a photopolymerizable composition according to claim 14, at least one ethylenically unsaturated compounds in amounts between about 0.1 and about 15 percent by weight and additives,
wherein said ethylenically unsaturated compounds are selected from one or more of:
polyurethane prepolymers, polyether polyisocyanate prepolymers;
epoxy oligomers: epoxy acrylate oligomers, epoxy polystyrene oligomers;
amino polymers: amino acrylate polymers or p-amino benzaldehyde polymers;
said additives can be silk screen inks, offset printing inks, flexo inks, wood coatings.

16. The use of the photoinitiation system according to claim 15, **characterized in that** it will produce cured compounds with properties of paint, varnish, enamel, pigment or ink by photopolymerization.

17. The use of the photoinitiation system according to claim 15, **characterized in that** it can be used as oily or water borne coating agents.
